(19) Europäisches Patentamt
European Patent Office
Office européen des brevets

(11) EP 3 897 145 B1

(12) **EUROPEAN PATENT SPECIFICATION**

(45) Date of publication and mention of the grant of the patent:
**04.06.2025 Bulletin 2025/23**

(21) Application number: **19832334.7**

(22) Date of filing: **17.12.2019**

(51) International Patent Classification (IPC):
***A01N 35/02*** *(2006.01)* ***A61K 8/35*** *(2006.01)*
***A01P 1/00*** *(2006.01)*

(52) Cooperative Patent Classification (CPC):
(C-Sets available)
**A01N 35/02; A61K 8/347; A61K 8/35; A61K 8/375; A61K 8/4973; A61Q 17/005** (Cont.)

(86) International application number:
**PCT/EP2019/085792**

(87) International publication number:
**WO 2020/127378 (25.06.2020 Gazette 2020/26)**

(54) **ANTIMICROBIAL MIXTURE CONTAINING 4-(3-ETHOXY-4-HYDROXYPHENYL)BUTAN-2-ONE AND AN ALCOHOL COMPOUND, AND COSMETIC COMPOSITION CONTAINING SAME**

ANTIMIKROBIELLE MISCHUNG MIT 4-(3-ETHOXY-4-HYDROXYPHENYL)BUTAN-2-ON UND EINER AMMONIUMVERBINDUNG UND KOSMETISCHE ZUSAMMENSETZUNG DAMIT

MÉLANGE ANTIMICROBIEN CONTENANT DE LA 4-(3-ÉTHOXY-4-HYDROXYPHÉNYL) BUTAN-2-ONE ET UN COMPOSÉ ALCOOL, ET COMPOSITION COSMÉTIQUE LE CONTENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2018 FR 1873695**

(43) Date of publication of application:
**27.10.2021 Bulletin 2021/43**

(73) Proprietor: **L'OREAL**
**75008 Paris (FR)**

(72) Inventors:
- **MENARD-SZCZEBARA, Florence**
**94152 CHEVILLY LA RUE (FR)**
- **CUPFERMAN, Sylvie**
**94152 CHEVILLY LA RUE (FR)**
- **GALVAN, Julien**
**94152 CHEVILLY LA RUE (FR)**

(74) Representative: **L'Oreal**
**Service D.I.P.I.**
**9, rue Pierre Dreyfus**
**92110 Clichy (FR)**

(56) References cited:
**EP-A1- 0 919 125**
**EP-A1- 2 879 492**
**WO-A1-2011/039445**
**JP-A- 2015 000 856**
**US-A1- 2004 214 785**
**US-A1- 2017 204 351**
**US-A1- 2018 333 494**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A01N 35/02, A01N 31/08, A01N 31/16, A01N 35/04, A01N 37/12, A01N 43/08**

## Description

The present invention relates to an antimicrobial mixture containing 4-(3-ethoxy-4-hydroxyphenyl)butan-2-one and an alcohol compound, and also to a cosmetic, pharmaceutical or nutritional composition containing such a mixture.

## Technical field

4-(3-Ethoxy-4-hydroxyphenyl)butan-2-one (ketone compound) is a beneficial substance as a preserving agent for cosmetic compositions, for protecting the compositions against microbial contamination, as described in patent application WO 2011/039445.

However, it is desirable to be able to incorporate said ketone compound in reduced concentration in compositions, notably cosmetic or dermatological compositions, while at the same time maintaining good antimicrobial conservation performance. Combinations of the ketone compound with other compounds that have good antimicrobial efficacy are thus sought for this purpose.

The invention may be better understood on reading the following description accompanied with nonlimiting implementation examples thereof with reference to the appended drawings, in which:

The inventors have discovered, unexpectedly, that the combination of

- at least one 4-(3-ethoxy-4-hydroxyphenyl)butan-2-one and salts thereof with an organic or inorganic acid or base, and solvates thereof such as the hydrates ; with
- at least one alcohol compound chosen from :
  - hydroxyacetophenone and salts thereof with an organic or inorganic acid or base, and solvates thereof such as the hydrates;
  - 5-chloro-2-(2,4-dichlorophenoxy)phenol and salts thereof with an organic or inorganic acid or base, and solvates thereof such as the hydrates;
  - sorbitan caprylate and its optical isomers, and solvates thereof such as the hydrates ;
  - the combination of :

    a) 80% to 90% by weight, relative to the total weight of said combination, of xylityl sesquicaprylate and its optical isomers, and solvates thereof such as the hydrates, and

    b) from 10% to 20% by weight of anhydroxylitol (especially 1,4-anhydroxylitol) and its optical isomers, and solvates thereof such as the hydrates ;
  - 4-chloro-3,5-dimethylphenol and salts thereof with an organic or inorganic acid or base, and solvates thereof such as the hydrates, and
  - glyceryl caprylate and its optical isomers, and solvates thereof such as the hydrates,

makes it possible to obtain an antimicrobial mixture which has synergistic antimicrobial activity.

More particularly, the combination of

- 4-(3-ethoxy-4-hydroxyphenyl)butan-2-one ; with
- an alcohol compound chosen from :
  - hydroxyacetophenone preferably 4-hydroxyacetophenone ;
  - 5-chloro-2-(2,4-dichlorophenoxy)phenol;
  - sorbitan caprylate ;
  - the combination of 80% to 90% by weight, relative to the total weight of said combination, of xylityl sesquicaprylate

and from 10% to 20% by weight of anhydroxylitol (especially 1,4-anhydroxylitol);

- 4-chloro-3,5-dimethylphenol, and
- glyceryl caprylate,

makes it possible to obtain an antimicrobial mixture which has synergistic antimicrobial activity.

The results of the examples described below show the synergistic antimicrobial activity obtained with the minimum inhibitory concentration (MIC) measurements taken with several mixtures. The antimicrobial activity is considered as being synergistic when the antimicrobial mixture makes it possible to obtain a percentage of strain growth of less than or equal to 25%, or even less than or equal to 20%.

Particularly, the combination of :

- 4-(3-ethoxy-4-hydroxyphenyl)butan-2-one and salts thereof with an organic or inorganic base, and solvates thereof such as the hydrates, especially 4-(3-ethoxy-4-hydroxyphenyl)butan-2-one ; with
- hydroxyacetophenone and salts thereof with an organic or inorganic base, and solvates thereof such as the hydrates, especially hydroxyacetophenone;

makes it possible to obtain an antimicrobial mixture with synergistic antimicrobial activity, in particular on molds, notably on *Aspergillus niger.*

According to one embodiment, the combination of :

- 4-(3-ethoxy-4-hydroxyphenyl)butan-2-one and salts thereof with an organic or inorganic base, and solvates thereof such as the hydrates, especially 4-(3-ethoxy-4-hydroxyphenyl)butan-2-one; with
- 5-chloro-2-(2,4-dichlorophenoxy)phenol and salts thereof with an organic or inorganic base, and solvates thereof such as the hydrates, especially 5-chloro-2-(2,4-dichlorophenoxy)phenol;

makes it possible to obtain an antimicrobial mixture with synergistic antimicrobial activity, in particular on the Gram-positive bacterium *Staphylococcus aureus.*

According to another embodiment, the combination of :

- 4-(3-ethoxy-4-hydroxyphenyl)butan-2-one and salts thereof with an organic or inorganic base, and solvates thereof such as the hydrates, especially 4-(3-ethoxy-4-hydroxyphenyl)butan-2-one; with
- sorbitan caprylate and its optical isomers, and solvates thereof such as the hydrates, especially sorbitan caprylate ;

makes it possible to obtain an antimicrobial mixture which has synergistic antimicrobial activity, in particular on molds, notably on *Aspergillus niger,* and on the Gram-positive bacterium *Enterococcus faecalis.*

According to another embodiment, the combination of :

- 4-(3-ethoxy-4-hydroxyphenyl)butan-2-one and salts thereof with an organic or inorganic base, and solvates thereof such as the hydrates, especially 4-(3-ethoxy-4-hydroxyphenyl)butan-2-one; with
- the combination of 80% to 90% by weight, relative to the total weight of said combination, of a) xylityl sesquicaprylate and its optical isomers, and solvates thereof such as the hydrates, especially xylityl sesquicaprylate, and b) from 10% to 20% by weight of anhydroxylitol (especially 1,4-anhydroxylitol) and its optical isomers, and solvates thereof such as the hydrates, particularly anhydroxylitol (especially 1,4-anhydroxylitol);

makes it possible to obtain an antimicrobial mixture with synergistic antimicrobial activity, in particular on the Gram-positive bacterium *Enterococcus faecalis,* on Gram-negative bacteria, notably on *Pseudomonas aeruginosa,* and on molds, notably on *Aspergillus niger.*

According to another embodiment, the combination of :

- 4-(3-ethoxy-4-hydroxyphenyl)butan-2-one and salts thereof with an organic or inorganic base, and solvates thereof such as the hydrates, especially 4-(3-ethoxy-4-hydroxyphenyl)butan-2-one; with
- 4-chloro-3,5-dimethylphenol and salts thereof with an organic or inorganic base, and solvates thereof such as the

hydrates, especially 4-chloro-3,5-dimethylphenol;

makes it possible to obtain an antimicrobial mixture which has synergistic antimicrobial activity, in particular on yeasts, in particular on *Candida albicans,* and on molds, notably on *Aspergillus niger.*

According to another embodiment, the combination of :

- 4-(3-ethoxy-4-hydroxyphenyl)butan-2-one and salts thereof with an organic or inorganic base, and solvates thereof such as the hydrates, especially 4-(3-ethoxy-4-hydroxyphenyl)butan-2-one ; with
- glyceryl caprylate and its optical isomers, and solvates thereof such as the hydrates, especially glyceryl caprylate ;

makes it possible to obtain an antimicrobial mixture which has synergistic antimicrobial activity, in particular on Gram-positive bacteria, notably on *Enterococcus faecalis* and *Staphylococcus aureus* and on Gram-negative bacteria, notably on *Pseudomonas aeruginosa.*

More precisely, a subject of the invention is an antimicrobial mixture comprising, or constituted of (or consisting of):

- 4-(3-ethoxy-4-hydroxyphenyl)butan-2-one and salts thereof with an organic or inorganic acid or base, and solvates thereof such as the hydrates; and
- an alcohol compound chosen from :
  - hydroxyacetophenone (preferably 4-hydroxyacetophenone) and salts thereof with an organic or inorganic base, and solvates thereof such as the hydrates,
  - 5-chloro-2-(2,4-dichlorophenoxy)phenol and salts thereof with an organic or inorganic base, and solvates thereof such as the hydrates,
  - sorbitan caprylate and its optical isomers, and solvates thereof such as the hydrates,
  - the combination of 80% to 90% by weight, relative to the total weight of said combination, of xylityl sesquicaprylate and its optical isomers, and solvates thereof such as the hydrates and from 10% to 20% by weight of anhydroxylitol (especially 1,4-anhydroxylitol) and its optical isomers, and solvates thereof such as the hydrates,
  - 4-chloro-3,5-dimethylphenol and salts thereof with an organic or inorganic base, and solvates thereof such as the hydrates, and
  - glyceryl caprylate and its optical isomers, and solvates thereof such as the hydrates.

For the purposes of the present invention, and unless otherwise indicated:

an *"organic or inorganic acid salt"* is more particularly chosen from salts chosen from a salt derived from i) hydrochloric acid HCl, ii) hydrobromic acid HBr, iii) sulfuric acid $H_2SO_4$, iv) alkylsulfonic acids: Alk-$S(O)_2OH$ such as methanesulfonic acid and ethanesulfonic acid; v) arylsulfonic acids: Ar-$S(O)_2OH$ such as benzenesulfonic acid and toluenesulfonic acid; vi) citric acid; vii) succinic acid; viii) tartaric acid; ix) lactic acid; x) alkoxysulfinic acids: Alk-O-S(O)OH such as methoxysulfinic acid and ethoxysulfinic acid; xi) aryloxysulfinic acids such as tolueneoxysulfinic acid and phenoxysulfinic acid; xii) phosphoric acid $H_3PO_4$; xiii) acetic acid $CH_3C(O)OH$; xiv) triflic acid $CF_3SO_3H$; and xv) tetrafluoroboric acid $HBF_4$;

an *"organic or inorganic base salts"* means salts of bases or alkaline agents as defined below, such as alkali metal hydroxides, such as sodium hydroxide, potassium hydroxide, or alkaline earth metal calcium hydroxide, ammonia, amines or alkanolamines.

an *"hydroxyacetophenone"* means 2-hydroxyacetophenone, 3-hydroxyacetophenone or 4-hydroxyacetophenone, preferably 4-hydroxyacetophenone;

an *"alkyl radical"* is a linear or branched hydrocarbonyl chain saturated;

moreover, the addition salts that may be used in the context of the invention with 4-(3-ethoxy-4-hydroxyphenyl) butan-2-one are especially chosen from addition salts with a cosmetically acceptable base such as basifying agents as defined below, for instance alkali metal hydroxides such as sodium hydroxide, potassium hydroxide, aqueous ammonia, amines or alkanolamines; preferably 4-(3-éthoxy-4-hydroxyphényl)butan-2-one can be in a salt form such as formula (I'):

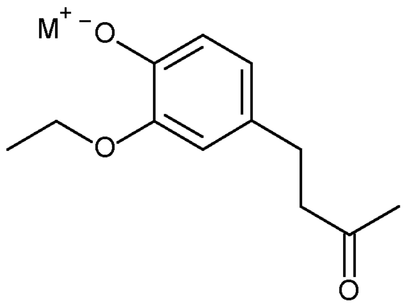

(I')

Formula (I') in which $M^+$ represents a cationic counter ion especially an alkali metal such as sodium or potassium, or alkaline earth metal such as calcium or ammonium. the term *"at least one"* is equivalent to the term *"one or more"*.

Another subject of the invention is a composition preferably a cosmetic, pharmaceutical or nutritional composition, in a physiologically acceptable medium, comprising:

- at least one 4-(3-ethoxy-4-hydroxyphenyl)butan-2-one and salts thereof with an organic or inorganic base, and solvates thereof such as the hydrates ; and
- at least one alcohol compound chosen from :
    - hydroxyacetophenone (preferably 4-hydroxyacetophenone) and salts thereof with an organic or inorganic base, and solvates thereof such as the hydrates
    - 5-chloro-2-(2,4-dichlorophenoxy)phenol and salts thereof with an organic or inorganic base, and solvates thereof such as the hydrates ;
    - sorbitan caprylate and its optical isomers, and solvates thereof such as the hydrates ;
    - the combination of 80 % to 90 % by weight, relative to the total weight of said combination, of xylityl sesquicaprylate and its optical isomers, and solvates thereof such as the hydrates and from 10% to 20% by weight of anhydroxylitol (especially 1,4-anhydroxylitol) and its optical isomers, and solvates thereof such as the hydrates;
    - 4-chloro-3,5-dimethylphenol and salts thereof with an organic or inorganic base, and solvates thereof such as the hydrates; and
    - glyceryl caprylate and its optical isomers, and solvates thereof such as the hydrates.

A subject of the invention is also a composition comprising said antimicrobial mixture.
The composition may comprise a physiologically acceptable medium. The composition is notably a cosmetic or pharmaceutical or dermatological composition.
The composition may optionally be a nutritional composition (food).
A subject of the invention is also a process for the nontherapeutic cosmetic treatment of keratin materials, comprising the application to the keratin materials of a composition, notably a cosmetic composition, as described previously. The process may be a cosmetic process for caring for or making up or cleansing keratin materials.
A subject of the invention is also a process for conserving a composition, notably comprising a physiologically acceptable medium, in particular a cosmetic or pharmaceutical composition, or a nutritional composition, characterized in that it consists in incorporating into said composition an antimicrobial mixture as described previously.
4-(3-Ethoxy-4-hydroxyphenyl)butan-2-one is a compound of formula:

[Chem. 1]

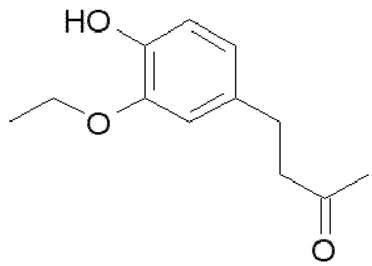

and salts thereof with an organic or inorganic base, and solvates thereof such as the hydrates.

According to a first embodiment, a subject of the invention is an antimicrobial mixture comprising, or constituted of (or consisting of), 4-(3-ethoxy-4-hydroxyphenyl)butan-2-one and hydroxyacetophenone.

Advantageously, 4-(3-ethoxy-4-hydroxyphenyl)butan-2-one and hydroxyacetophenone are present in said mixture in a content such that the 4-(3-ethoxy-4-hydroxyphenyl)butan-2-one/hydroxyacetophenone weight ratio ranges from 0.08 to 0.5, preferably ranges from 0.08 to 0.3, preferentially ranges from 0.08 to 0.25 and more preferentially ranges from 0.15 to 0.25. Such a mixture has good antimicrobial activity on molds, notably on *Aspergillus niger.*

Preferably, the composition comprising the antimicrobial mixture may comprise 4-(3-ethoxy-4-hydroxyphenyl) butan-2-one in a content ranging from 0.02 % to 0.09 % by weight, preferentially ranging from 0.03 % to 0.75 % by weight and better still ranging from 0.04 % to 0.06 % by weight, relative to the total weight of the composition.

According to a second embodiment, a subject of the invention is an antimicrobial mixture comprising, or constituted of (or consisting of), 4-(3-ethoxy-4-hydroxyphenyl)butan-2-one and 5-chloro-2-(2,4-dichlorophenoxy)phenol.

The antimicrobial mixture may have a 4-(3-ethoxy-4-hydroxyphenyl)butan-2-one/5-chloro-2-(2,4-dichlorophenoxy)phenol weight ratio ranging from 0.3 to 6, preferably ranging from 0.7 to 6, preferentially ranging from 1.5 to 6 and more preferentially ranging from 3 to 6. Such a mixture has good antimicrobial activity on the Gram-positive bacterium *Staphylococcus aureus.*

Preferably, the composition comprising the antimicrobial mixture may comprise 4-(3-ethoxy-4-hydroxyphenyl) butan-2-one in a content ranging from 0.05% to 0.9% by weight, relative to the total weight of the composition, preferably ranging from 0.08 % to 0.9 % by weight, preferably ranging from 0.1% to 0.9% by weight, preferably ranging from 0.15 % to 0.9 % by weight, preferably ranging from 0.2 % to 0.9 % by weight, preferably ranging from 0.3 % to 0.9 % by weight, preferably ranging from 0.4 % to 0.8 % by weight, preferably ranging from 0.4 % to 0.7 % by weight.

According to a third embodiment, a subject of the invention is an antimicrobial mixture comprising, or constituted of (or consisting of), 4-(3-ethoxy-4-hydroxyphenyl)butan-2-one and sorbitan caprylate.

Sorbitan caprylate is a monoester (CAS No. 95508-00-2); it is notably sold under the name Velsan SC by the company Clariant.

Advantageously, 4-(3-ethoxy-4-hydroxyphenyl)butan-2-one and sorbitan caprylate are present in said mixture in a content such that the 4-(3-ethoxy-4-hydroxyphenyl)butan-2-one/sorbitan caprylate weight ratio ranges from 0.1 to 3.2, preferably from 0.2 to 1.6 and preferably from 0.4 to 1.4.

The antimicrobial mixture may have a 4-(3-ethoxy-4-hydroxyphenyl)butan-2-one/sorbitan caprylate weight ratio ranging from 0.4 to 1.6, preferably from 0.6 to 1.6 and preferentially from 0.8 to 1.4. Such a mixture has good antimicrobial activity on molds, notably on *Aspergillus niger.* Preferably, the composition comprising the antimicrobial mixture may comprise 4-(3-ethoxy-4-hydroxyphenyl)butan-2-one in a content ranging from 0.02 % to 0.09 % by weight, preferentially ranging from 0.02 % to 0.08 % by weight, better still ranging from 0.02% to 0.07 % by weight and even better still ranging from 0.02 % to 0.06 % by weight, relative to the total weight of the composition.

The antimicrobial mixture may have a 4-(3-ethoxy-4-hydroxyphenyl)butan-2-one/sorbitan caprylate weight ratio ranging from 0.1 to 3.2, preferably from 0.2 to 1.6 and preferentially from 0.4 to 1.2. Such a mixture has good antimicrobial activity on the Gram-positive bacterium *Enterococcus faecalis.* Preferably, the composition comprising the antimicrobial mixture may comprise 4-(3-ethoxy-4-hydroxyphenyl)butan-2-one in a content ranging from 0.05 % to 0.04 % by weight, preferentially ranging from 0.05 % to 0.3 % by weight, relative to the total weight of the composition.

According to a fourth embodiment, a subject of the invention is an antimicrobial mixture comprising, or constituted of (or consisting of) 4-(3-ethoxy-4-hydroxyphenyl)butan-2-one and a combination of 80 % to 90 % by weight, relative to the total weight of said combination, of xylityl sesquicaprylate and from 10 % to 20 % by weight of anhydroxylitol (also referred to hereinbelow as the xylityl sesquicaprylate/anhydroxylitol combination).

Xylityl sesquicaprylate is a mixture of caprylic acid monoesters and diesters of xylitol; its CAS No. is 181632-90-6. Anhydroxylitol has the CAS No. 53448-53-6.

Use may be made of the combination of xylityl sesquicaprylate and anhydroxylitol (85/15 weight/weight) sold under the name Hebeatol Plus from Chemyunion (INCI name: xylityl sesquicaprylate (and) anhydroxylitol).

The antimicrobial mixture may have a 4-(3-ethoxy-4-hydroxyphenyl)butan-2-one/combination of xylityl sesquicaprylate/anhydroxylitol weight ratio ranging from 0.2 to 13, preferably ranging from 0.2 to 10, preferably ranging from 0.2 to 8, more preferentially ranging from 0.4 to 5, preferably 0.6 to 3. Such a mixture has good antimicrobial activity on Gram-negative bacteria, notably on *Pseudomonas aeruginosa.* Preferably, the composition comprising the antimicrobial mixture may comprise 4-(3-ethoxy-4-hydroxyphenyl)butan-2-one in a content ranging from 0.05 % to 0.9 % by weight, preferably from 0.08 % to 0.9 % by weight, preferably from 0.1 % to 0.9 % by weight, preferably from 0.2 % to 0.9 % by weight, preferably from 0.35 % to 0.75 % by weight, relative to the total weight of the composition.

The antimicrobial mixture may have a 4-(3-ethoxy-4-hydroxyphenyl)butan-2-one/combination of xylityl sesquicaprylate/anhydroxylitol weight ratio ranging from 0.4 to 3.2, preferably from 0.6 to 3, preferably from 1.5 to 2.5. Such a mixture has good antimicrobial activity on the Gram-positive bacterium Enterococcus faecalis. Preferably, the composition comprising the antimicrobial mixture may comprise 4-(3-ethoxy-4-hydroxyphenyl)butan-2-one in a content ranging from 0.05 % to 0.4 % by weight, preferably from 0.08 % to 0.4 % by weight, preferably from 0.1 % to 0.4 % by weight, preferably from 0.15 % to 0.4 % by weight, preferably from 0.15 % to 0.35 % by weight, relative to the total weight of the composition.

According to a fifth embodiment, a subject of the invention is an antimicrobial mixture comprising, or constituted of (or consisting of), 4-(3-ethoxy-4-hydroxyphenyl)butan-2-one and 4-chloro-3,5-dimethylphenol.

The antimicrobial mixture may have a 4-(3-ethoxy-4-hydroxyphenyl)butan-2-one/4-chloro-3,5-dimethylphenol weight ratio ranging from 0.1 to 1.4, preferably ranging from 0.15 to 1.4, preferably ranging from 0.15 to 1.2.

The antimicrobial mixture may have a 4-(3-ethoxy-4-hydroxyphenyl)butan-2-one/4-chloro-3,5-dimethylphenol weight ratio ranging from 0.15 to 1.4, preferably from 0.25 to 1.4, preferably from 0.5 to 1.2. Such a mixture has good antimicrobial activity on yeasts, in particular on *Candida albicans.* Preferably, the composition comprising the antimicrobial mixture may comprise 4-(3-ethoxy-4-hydroxyphenyl)butan-2-one in a content ranging from 0.02 % to 0.15 % by weight, preferably from 0.04 % to 0.15 % by weight, preferably from 0.07 % to 0.15 % by weight, preferably from 0.08 % to 0.12 % by weight, relative to the total weight of the composition.

The antimicrobial mixture may have a 4-(3-ethoxy-4-hydroxyphenyl)butan-2-one/4-chloro-3,5-dimethylphenol weight ratio ranging from 0.1 to 0.7, preferably from 0.15 to 0.5, preferably from 0.15 to 0.35, preferably from 0.15 to 0.25, preferably from 0.3 to 0.7, preferably from 0.3 to 0.5. Such a mixture has good antimicrobial activity on molds, notably on *Aspergillus niger.* Preferably, the composition comprising the antimicrobial mixture may comprise 4-(3-ethoxy-4-hydroxyphenyl)butan-2-one in a content ranging from 0.035 % to 0.09 % by weight, preferably from 0.035 % to 0.08 % by weight, preferably from 0.04 % to 0.07 % by weight, preferably from 0.04 % to 0.06 % by weight, relative to the total weight of the composition.

According to a sixth embodiment, a subject of the invention is an antimicrobial mixture comprising, or constituted of (or consisting of), 4-(3-ethoxy-4-hydroxyphenyl)butan-2-one and glyceryl caprylate.

Glyceryl caprylate is a monoester (CAS No. 26402-26-6).

The antimicrobial mixture may have a 4-(3-ethoxy-4-hydroxyphenyl)butan-2-one/glyceryl caprylate weight ratio ranging from 0.1 to 10. Such a mixture has good antimicrobial activity on the Gram-positive bacterium *Staphylococcus aureus.* Preferably, the composition comprising the antimicrobial mixture may comprise 4-(3-ethoxy-4-hydroxyphenyl)butan-2-one in a content ranging from 0.05 % to 0.9 % by weight, relative to the total weight of the composition, preferably ranging from 0.08 % to 0.9 % by weight, preferably ranging from 0.1 % to 0.9 % by weight, preferably ranging from 0.15 % to 0.9 % by weight, preferably ranging from 0.2 % to 0.9 % by weight, preferably ranging from 0.3 % to 0.9 % by weight, preferably ranging from 0.4 % to 0.8 % by weight, preferably ranging from 0.4 % to 0.7 % by weight.

The antimicrobial mixture may have a 4-(3-ethoxy-4-hydroxyphenyl)butan-2-one/glyceryl caprylate weight ratio ranging from 0.5 to 9, preferably ranging from 0.5 to 7, preferably from 1 to 6 and preferentially from 1 to 3. Such a mixture has good antimicrobial activity on Gram-negative bacteria, notably on *Pseudomonas aeruginosa.* Preferably, the composition comprising the antimicrobial mixture may comprise 4-(3-ethoxy-4-hydroxyphenyl)butan-2-one in a content ranging from 0.08 % to 0.9 % by weight, preferably ranging from 0.1 % to 0.9 % by weight, preferably ranging from 0.2 % to

0.9 % by weight, preferably ranging from 0.35 % to 0.75 % by weight, relative to the total weight of the composition.

The antimicrobial mixture may have a 4-(3-ethoxy-4-hydroxyphenyl)butan-2-one/glyceryl caprylate weight ratio ranging from 0.2 to 5, preferably ranging from 0.9 to 4.5. Such a mixture has good antimicrobial activity on the Gram-positive bacterium *Enterococcus faecalis*. Preferably, the composition comprising the antimicrobial mixture may comprise 4-(3-ethoxy-4-hydroxyphenyl)butan-2-one in a content ranging from 0.05 % to 0.4 % by weight, preferably from 0.08 % to 0.4 % by weight, preferably from 0.1 % to 0.4 % by weight, preferably from 0.15 % to 0.4 % by weight, preferably from 0.15 % to 0.35 % by weight, relative to the total weight of the composition.

A subject of the invention is also a composition comprising, in a physiologically acceptable medium, the antimicrobial mixture described previously.

The term "physiologically acceptable medium" means a medium that is compatible with human keratin materials such as the skin, the scalp, the hair and the nails. Said medium may comprise one or more additional ingredients other than the ketone compound and the alcohol compound.

The compound 4-(3-ethoxy-4-hydroxyphenyl)butan-2-one may be present in the composition according to the invention in a content ranging from 0.01 % to 5 % by weight, relative to the total weight of the composition, preferably ranging from 0.01 % to 3 % by weight, preferentially ranging from 0.01 % to 2.5 % by weight, more preferentially ranging from 0.01 % to 2 % by weight and better still ranging from 0.02 % to 0.15 % by weight, or else in the contents described previously.

The composition may comprise at least one additional ingredient chosen from water, oils, polyols containing from 2 to 10 carbon atoms, gelling agents, surfactants, film-forming polymers, dyestuffs, fragrances, fillers, UV-screening agents, plant extracts, cosmetic and dermatological active agents, and salts.

The composition according to the invention may comprise an aqueous phase.

The composition may also comprise a polyol that is water-miscible at room temperature (25°C), notably chosen from polyols notably containing from 2 to 10 carbon atoms, preferably containing from 2 to 6 carbon atoms, such as glycerol, propylene glycol, 1,3-propanediol, butylene glycol, pentylene glycol, hexylene glycol, dipropylene glycol, diethylene glycol or diglycerol. Advantageously, the composition according to the invention comprises 1,3-propanediol, notably in a content ranging from 0.1% to 20% by weight, preferably ranging from 0.1% to 10% by weight and preferentially ranging from 0.5% to 5% by weight, relative to the total weight of the composition.

The compositions according to the invention may be in the form of oil-in-water (O/W) emulsions, water-in-oil (W/O) emulsions or multiple emulsions (triple: W/O/W or O/W/O), oily solutions, oily gels, aqueous solutions, aqueous gels, or solid compositions. These compositions are prepared according to the usual methods.

The compositions according to the invention may be more or less fluid and may have the appearance of a white or coloured cream, an ointment, a milk, a lotion, a serum, a paste or a foam. They may be optionally applied to the skin in aerosol form. They may also be in solid form, for example in the form of a stick or a compact powder.

The composition according to the invention may notably be in the form of:

- a makeup product, notably for making up the skin of the face, the body, or the lips or the eyelashes;
- an aftershave gel or lotion; a shaving product;
- a deodorant (stick, roll-on or aerosol);
- a hair-removing cream;
- a body hygiene composition such as a shower gel or a shampoo;
- a pharmaceutical composition;
- a solid composition such as a soap or a cleansing bar;
- an aerosol composition also comprising a pressurized propellant;
- a hair-setting lotion, a hair-styling cream or gel, a dyeing composition, a permanent-waving composition, a lotion or a gel for combating hair loss, or a hair conditioner;
- a composition for caring for or cleansing the skin.

A subject of the invention is also a process for preparing a composition, notably a cosmetic or pharmaceutical or nutritional composition, comprising a step of mixing 4-(3-ethoxy-4-hydroxyphenyl)butan-2-one, the alcohol compound and one or more additional ingredients, notably cosmetic or pharmaceutical or nutritional ingredients, such as those described previously.

The antimicrobial mixtures of the fourth, fifth and sixth embodiments described previously may be used for preserving foodstuffs (food).

A subject of the invention is also a nutritional composition comprising an antimicrobial mixture chosen from those of the fourth, fifth and sixth embodiments described previously.

The nutritional composition (food) may comprise at least one foodstuff chosen from meats, fish, crustaceans, vegetables, fruit, cereals, eggs, butter, milk, vinegar, water, vegetable oils, sugars, salt, spices, emulsifiers, alcohols,

thickeners and honey.

Preferably the 4-(3-ethoxy-4-hydroxyphenyl)butan-2-one is predissolved in a food-grade solvent before being added to the food. Solvents that may be used include ethanol, propylene glycol, isopropyl alcohol and mixtures thereof, optionally combined with water.

The food according to the invention may be, for example, in the form of bread, cake, sauce, candy, a cooked dish, confectionery, jelly, dessert, nougat, drinks, juice, syrup, wine, beer, ravioli, mousse, compote, mayonnaise, mustard, vinaigrette, crisps, sausage, gnocchi, polenta, pancakes, pâtés, cheeses, flour, delicatessen meats or soup.

The 4-(3-ethoxy-4-hydroxyphenyl)butan-2-one may be present in the food composition in the concentrations described previously.

The synergistic activity of the antimicrobial mixture according to the invention on Gram-negative bacteria, in particular on bacteria of the species *Pseudomonas aeruginosa* is also of interest in water treatment. This species is notably among the microorganisms commonly sought when a microbiological analysis of a water sample is performed.

The synergistic activity of the antimicrobial mixture according to the invention on fungi, in particular on the species *Candida albicans,* is also of interest in water treatment. Specifically, fungi represent one of the sources of contamination of water as mentioned in the article "Fungal Contaminants in drinking water regulation? A tale of ecology, exposure, purification and clinical relevance" Int. J. Environ. Res. Public Health 2017, 14, 636.

The synergistic activity of the antimicrobial mixture according to the invention on Gram-positive bacteria, in particular on bacteria of the species *Enterococcus faecalis* is also of interest in water treatment. Specifically, bacteria of the genus *Enterococcus* represent one of the sources of contamination of water as mentioned in the article "Enterococci, from commensals to leading causes of drug resistant infection" Michael S. Gilmore et al., 2014, Massachusetts Eye and Ear Infirmary, Boston.

The antimicrobial mixtures of the third, fourth, fifth and sixth embodiments described previously may be used for water treatment.

The present invention also relates to the use of the antimicrobial mixture chosen from those of the third, fourth, fifth and sixth embodiments described previously in water treatment, in which said water is chosen from domestic or industrial waters, waters from aquatic media, swimming pool/spa waters, and waters from air-conditioning systems.

The term "water treatment" refers to a continuous or discontinuous (batch-type) treatment which consists in adding a substance to a water sample to be treated or to a water stream to be treated for the purpose either of preventing the contamination of the water with a contaminant or of partially or totally decontaminating of said contaminant said water to be treated

Preferably, the water treatment performed in the context of the present invention consists in continuously or discontinuously adding a substance to a sample of water to be treated or to a water stream to be treated in order to partially or totally decontaminate of a contaminant said water to be treated.

The contaminant may be a microorganism, in particular a bacterium and/or a fungus.

Even more preferentially, said water treatment is a treatment of water contaminated with one or more microorganisms, preferably with Gram-positive or Gram-negative bacteria or fungi of the species *Enterococcus faecalis, Candida albicans* or *Pseudomonas aeruginosa.*

The term "waters of aquatic media" means the waters of lakes, tributary rivers, pools, mainstem rivers, sea or ocean bathing areas, underground waters such as well waters and groundwaters, and aquarium waters.

For the purposes of the present invention, the "domestic or industrial waters" comprise spent waters before they have been treated in a purification plant, waters undergoing treatment in a purification plant, waters before they have been treated in a drinking water plant, waters undergoing treatment in a drinking water plant, and also waters circulating in potable or non-potable urban networks, for instance waters circulating in pipeworks.

The present invention also relates to a continuous or discontinuous water treatment process comprising at least one step of placing a water sample to be treated or a water stream to be treated, said water to be treated being chosen from domestic or industrial waters, waters from aquatic media, swimming pool/spa waters, and waters from air-conditioning systems, in contact with the antimicrobial mixture according to the invention.

Preferably, said step of placing the water to be treated in contact with the antimicrobial mixture according to the invention may notably be performed by injection in liquid form of said compound, by passage through a filter or a filtering cartridge comprising said compound, or by administration in solid form of said compound notably in the form of granules, lozenges or pellets.

The 4-(3-ethoxy-4-hydroxyphenyl)butan-2-one may be used in a proportion of at least 0.06 % by weight, preferably at least 0.1% by weight and better still at least 0.5 % by weight relative to the total weight of water to be treated. In a preferred embodiment, the compounds of formula (I), alone or as a mixture, may be used in a proportion of at least 1 % by weight relative to the total weight of water to be treated.

The 4-(3-ethoxy-4-hydroxyphenyl)butan-2-one may be used in a concentration ranging from 0.06 % to 10 % by weight, preferably from 0.06 % to 10 % by weight and better still from 0.06 % to 5 % by weight relative to the total weight of water to be treated. In a preferred embodiment, the 4-(3-ethoxy-4-hydroxyphenyl)butan-2-one may be used in a

concentration ranging from 0.1 % to 1 % by weight relative to the total weight of water to be treated.

In a preferred embodiment, the 4-(3-ethoxy-4-hydroxyphenyl)butan-2-one is used in combination with an effective amount of at least one organic solvent which may be chosen from ethanol, 1,2-propylene glycol, 1,3-propanediol, PEG-8 (polyethylene glycol containing 8 ethylene glycol units), propylene carbonate, dipropylene glycol, 1,2-hexylene glycol, PEG-4.

Preferably, the organic solvent is chosen from ethanol, 1,2-propylene glycol, 1,3-propanediol, PEG-8 and propylene carbonate.

The solvent may be used in a content ranging from 0.05 % to 10 % by weight relative to the total weight of the water to be treated, preferably ranging from 0.1 % to 5 % by weight and preferentially ranging from 0.1 % to 2.5 % by weight relative to the total weight of the water to be treated.

The invention is illustrated in greater detail in the example that follows. The amounts of the ingredients are expressed as weight percentages.

**Examples**

**Example 1: Determination of the synergistic antimicrobial activity as MIC**

The demonstration of a synergistic antimicrobial activity effect with a mixture of 4-(3-ethoxy-4-hydroxyphenyl) butan-2-one (referred to as substance A) and of an alcohol compound (referred to as substance B) was performed by calculating the synergy index (or FIC index) according to the following formula:

[Math. 1]

$$\text{FIC Index} = (\text{MIC of A with B} / \text{MIC of A}) + (\text{MIC of B with A} / \text{MIC of B})$$

with:

- MIC of A with B: minimum concentration of product A in the combination A + B which makes it possible to obtain an inhibitory effect;
- MIC of B with A: minimum concentration of product B in the combination A + B which makes it possible to obtain an inhibitory effect;
- MIC of A: minimum inhibitory concentration of product A alone;
- MIC of B: minimum inhibitory concentration of product B alone.

This formula was described for the first time in the article by F.C. Kull, P.C. Eisman, H.D. Sylwestrowka, and R.L. Mayer, Applied Microbiology 9:538-541, 1961.

For each compound tested alone, the MIC is considered as the first concentration which makes it possible to obtain a microbial growth percentage of less than or equal to 25 %.

As regards the combinations tested, MIC of A with B and MIC of B with A are the respective concentrations of A and of B in the combinations which make it possible to obtain a microbial growth percentage of less than or equal to 25 %.

**Interpretation of the FIC Index:**

When the FIC index value is less than or equal to 1, it is considered that the combination of test compounds has a synergistic effect.

The summary of the results obtained is presented in the following tables.

The combination of compounds A and B was tested on the following strains or a part thereof: *Aspergillus niger, Staphylococcus aureus, Pseudomonas aeruginosa, Enterococcus faecalis, Candida albicans.*

The microbial strain *Aspergillus niger* ATCC 6275, and a double-concentration Sabouraud broth liquid culture medium supplemented with polyoxyethylenated (20 OE) sorbitan monopalmitate (Tween 40 from Croda) and Phytagel© BioReagent were used (i.e. a mixture of 5 g of Phytagel + 0.6 g of Tween 40 + 60 g of Sabouraud broth).

The microbial strain *Staphylococcus aureus* ATCC 6538 and a double-concentration nutrient broth liquid culture medium were used.

The microbial strain *Pseudomonas aeruginosa* ATCC 9027 and a double-concentration nutrient broth liquid culture medium were used.

The microbial strain *Enterococcus faecalis* ATCC 33186 and a double-concentration BHI (Brain Heart Infusion) broth liquid culture medium were used.

The microbial strain *Candida albicans* ATCC 10231, and a double-concentration Sabouraud broth liquid culture medium were used (i.e. a mixture of 5 g of Phytagel + 0.6 g Tween 40 + 60 g of Sabouraud broth).
A 96-well microplate at an incubation temperature of 32.5°C is used.
The incubation time of the microplate is:

- from 24 to 30 h under aerobic conditions for microbial *Aspergillus niger* ATCC 6275;
- from 18 to 24 h under aerobic conditions for *Candida albicans* ATCC 10231, *Pseudomonas aeruginosa* ATCC 9027 and *Staphylococcus aureus* ATCC 6538;
- from 24 to 48 h under aerobic conditions for *Enterococcus faecalis* ATCC 33186.

**Tests**

For each compound:

A = 4-(3-ethoxy-4-hydroxyphenyl) butan-2-one compound

B = alcohol compound

A 10 % (weight/volume) stock solution was prepared by mixing 1 g of compound in 9 ml of aqueous 1 ‰ agar solution. Successive dilutions were made with the 1 ‰ agar solution.

**Tests of compounds A and B alone**

50 µl of each of the daughter solutions obtained containing compound A or B are added to the microplate wells. 100 µl of Sabouraud liquid nutrient broth inoculated at double concentration with the *Aspergillus niger* strain and 50 µl of aqueous 1 ‰ agar solution are also added thereto.

**Tests of compounds A and B as a mixture**

50 µl of each of the daughter solutions obtained containing compound A and 50 µl of each of the daughter solutions obtained containing compound B are added to the microplate wells. 100 µl of Sabouraud liquid nutrient broth inoculated at double concentration with the strain *Aspergillus niger* are also added thereto.

**Microbial growth control**

A positive microbial growth control was also prepared. The positive microbial growth control corresponds to a mixture of 100 µl of aqueous 1 ‰ agar solution with 100 µl of Sabouraud liquid nutrient broth inoculated at double concentration with the strain *Aspergillus niger* in the absence of compounds A and B.

**Absorbance control for compounds A and B alone**

An absorbance control was performed in parallel on compounds A and B alone. This control corresponds to 100 µl of double concentration sterile Sabouraud liquid nutrient broth + 100 µl of double concentration compound A or B.
In the three cases (absorbance control, growth control and test), the final volume present in each of the microplate wells is 200 µl.
In the two cases (test and control), the inoculum represents the concentration of the *Aspergillus niger* strain present in the final volume of the wells (200 µl) and is between 2 and $6 \times 10^5$ cfu/ml of *Aspergillus niger.*
The minimum inhibitory concentration (MIC) of each compound A and B alone and in combination was determined in a known manner by means of optical density measurements at a wavelength of 620 nm.
The test as described above (tests, absorbance control and growth control) was performed again to test the combination A + B on the following strains *Enterococcus faecalis, Staphylococcus aureus, Pseudomonas aeruginosa, Candida albicans,* where appropriate.
The following results were obtained with compound B1 = 4-hydroxyacetophenone:

*Aspergilus niger*

[Tables 1]

| **Concentrations tested (in weight %)** | 0 A | 0.025 A | 0.05 A | **0.1 A** |
|---|---|---|---|---|
| 0 B1 | | 82 | 43 | 5 |
| 0.0625 B1 | 86 | 71 | 46 | 3 |
| 0.125 B1 | 72 | 73 | **20 (FIC 0.75)** | 2 |
| 0.25 B1 | 79 | **22 (FIC 0.75)** | **12 (FIC 1)** | 5 |
| **0.5 B1** | 6 | 2 (FIC 0.75) | -19 (FIC 1) | 6 |

[Tables 2]

| **% MIC of A alone** | **% MIC of B1 alone** | **MIC of each compound as a mixture** | | **FIC Index** | **Ratio A/B1** |
|---|---|---|---|---|---|
| | | **A %** | **B1 %** | | |
| 0.1 | 0.5 | 0.05 | 0.25 | 1 | 0.2 |

The results obtained show synergistic inhibitory activity for the mixtures:

0.05 % of A and 0.25% of B1, i.e. A/B1 ratio = 0.2

0.05 % of A and 0.125% of B1, i.e. A/B1 ratio = 0.4

0.025 % of A and 0.25% of B1, i.e. A/B1 ratio = 0.1

**Example 2: determination of the synergistic antimicrobial activity as MIC on *Staphylococcus aureus***

The demonstration of a synergistic antimicrobial activity effect with a mixture of 4-(3-ethoxy-4-hydroxyphenyl) butan-2-one (referred to as substance A) and of 5-chloro-2-(2,4-dichlorophenoxy)phenol (referred to as substance B2) was performed according to the conditions described in Example 1.

The following results were obtained:

***Staphylococcus aureus***

[Tables 3]

| Concentrations tested (in weight %) | 0 A | 0.0625 A | 0.125 A | 0.25 A | 0.5 A | **1 A** |
|---|---|---|---|---|---|---|
| 0 B2 | | 52 | 49 | 52 | 75 | 2 |
| 0.125 B2 | 46 | **-8 (FIC 0.56)** | **-4 (FIC 0.63)** | **-9 (FIC 0.75)** | **-12 (FIC 1)** | -1 |
| **0.25 B2** | 14 | -7 | 6 | 5 | -10 | -1 |

[Tables 4]

| % MIC of A alone | % MIC of B2 alone | MIC of each compound as a mixture | | FIC Index | Ratio A/B2 |
|---|---|---|---|---|---|
| | | A % | B2 % | | |
| 1 | 0.25 | 0.5 | 0.125 | 1 | 4 |

The results obtained show synergistic inhibitory activity for the mixtures:

i) 0.5% of A and 0.125% of B2, i.e. A/B2 ratio = 4

ii) 0.25% of A and 0.125% of B2, i.e. A/B2 ratio = 2

iii) 0.125% of A and 0.125% of B2, i.e. A/B2 ratio = 1

iv) 0.0635% of A and 0.125% of B2, i.e. A/B2 ratio = 0.5

**Example 3:**

The demonstration of a synergistic antimicrobial activity effect with a mixture of 4-(3-ethoxy-4-hydroxyphenyl) butan-2-one (referred to as substance A) and of sorbitan caprylate (referred to as substance B3) was performed according to the conditions described in Example 1.

***Aspergillus niger***

[Tables 5]

| Concentrations tested (in weight %) | 0 A | 0.025 A | 0.05 A | **0.1 A** |
|---|---|---|---|---|
| 0 B3 | | 45 | 35 | 14 |
| 0.025 B3 | 50 | 42 | 26 | 12 |
| 0.05 B3 | 30 | **19 (FIC 0.75)** | **15 (FIC 1)** | 5 |
| **0.1 B3** | -2 | 3 | 8 | 6 |

[Tables 6]

| % MIC of A alone | % MIC of B3 alone | MIC of each compound as a mixture | | FIC Index | Ratio A/B3 |
|---|---|---|---|---|---|
| | | A % | B3 % | | |
| 0.1 | 0.1 | 0.05 | 0.05 | 1 | 1 |

The results obtained show synergistic inhibitory activity for the mixtures:

i) 0.05 % of A and 0.05 % of B3, i.e. A/B3 ratio = 1

ii) 0.025 % of A and 0.05 % of B3, i.e. A/B3 ratio = 0.5

***Enteroccocus faecalis***

[Tables 7]

| Concentrations tested (in weight %) | 0 A | 0.0625 A | 0.125 A | 0.25 A | **0.5 A** |
|---|---|---|---|---|---|
| 0 B3 | | 70 | 31 | 27 | 14 |
| 0.125 B3 | 134 | 117 | 62 | **-16** | -1 |
| 0.25 B3 | 61 | **-85** | **4** | **2** | 4 |
| 0.5 B3 | 62 | **-8** | **0** | **-9** | -12 |
| **1 B3** | 2 | -8 | -5 | -1 | 3 |

[Tables 8]

| % MIC of A alone | % MIC of B3 alone | MIC of each compound as a mixture | | FIC Index | Ratio A/B3 |
|---|---|---|---|---|---|
| | | A % | B3 % | | |
| 0.5 | 1 | 0.25 | 0.5 | 1 | 0.5 |

The results obtained show synergistic inhibitory activity for the mixtures:

i) 0.25 % of A and 0.5 % of B3, i.e. A/B3 ratio = 0.5

ii) 0.125 % of A and 0.5 % of B3, i.e. A/B3 ratio = 0.25

iii) 0.0625 % of A and 0.5 % of B3, i.e. A/B3 ratio = 0.125

iv) 0.25 % of A and 0.25 % of B3, i.e. ratio A/B3 = 1

v) 0.125 % of A and 0.25 % of B3, i.e. ratio A/B3 = 0.5

vi) 0.0625 % of A and 0.25 % of B3, i.e. ratio A/B3 = 0.25

vii) 0.25 % of A and 0.125 % of B3, i.e. ratio A/B3 = 2

## Example 4:

The demonstration of a synergistic antimicrobial activity effect with a mixture of 4-(3-ethoxy-4-hydroxyphenyl) butan-2-one (referred to as substance A) and of the combination of xylityl sesquicaprylate and anhydroxylitol (85/15 weight/weight) sold under the name Hebeatol Plus from Chemyunion (referred to as substance B4) was performed according to the conditions described in Example 1.

***Pseudomonas aeruginosa***

[Tables 9]

| Concentrations tested (in weight %) | 0 A | 0.0625 A | 0.125 A | 0.25 A | 0.5 A | **1 A** |
|---|---|---|---|---|---|---|
| 0 B4 | | 63 | 60 | 64 | 62 | 7 |
| 0.0625 B4 | 100 | 60 | 27 | **12 (FIC 0.38)** | **-10 (FIC 0.5)** | -8 |
| 0.125 B4 | 57 | **15 (FIC 0.31)** | **-4 (FIC 0.38)** | **-9 (FIC 0.5)** | **3 (FIC 0.75)** | 0 |
| 0.25 B4 | 52 | **-5 (FIC 0.56)** | **-9 (FIC 0.63)** | **-23 (FIC 0.75)** | **-22 (FIC 1)** | -5 |
| **0.5 B4** | 20 | -5 | -13 | -11 | 7 | -3 |

[Tables 10]

| % MIC of A alone | % MIC of B4 alone | MIC of each compound as a mixture | | FIC Index | Ratio A/B4 |
|---|---|---|---|---|---|
| | | A % | B4 % | | |
| 1 | 0.5 | 0.5 | 0.25 | 1 | 2 |

The results obtained show synergistic inhibitory activity for the mixtures:

i) 0.5 % of A and 0.25 % of B4, i.e. A/B4 ratio = 2

ii) 0.25 % of A and 0.25 % of B4, i.e. A/B4 ratio = 1

iii) 0.125 % of A and 0.25 % of B4, i.e. A/B4 ratio = 0.5

iv) 0.0625 % of A and 0.25 % of B4, i.e. A/B4 ratio = 0.25

v) 0.5 % of A and 0.125 % of B4, i.e. ratio A/B4 = 4

vi) 0.25 % of A and 0.125 % of B4, i.e. ratio A/B4 = 2

vii) 0.125 % of A and 0.125 % of B4, i.e. ratio A/B4 = 1

viii) 0.0625 % of A and 0.125 % of B4, i.e. ratio A/B4 = 0.5

ix) 0.5 % of A and 0.0625 % of B4, i.e. ratio A/B4 = 8

x) 0.25 % of A and 0.0625 % of B4, i.e. ratio A/B4 = 4

***Enterococcus faecalis***

[Tables 11]

| Concentrations tested (in weight %) | 0 A | 0.0625 A | 0.125 A | 0.25 A | **0.5 A** |
|---|---|---|---|---|---|
| 0 B4 | | 44 | 39 | 38 | 12 |
| 0.0625 B4 | 45 | 53 | 41 | 30 | 5 |
| 0.125 B4 | 42 | **6 (FIC 0.63)** | **7 (FIC 0.75)** | **1 (FIC 1)** | 11 |

| | | | | | |
|---|---|---|---|---|---|
| **0.25 B4** | -6 | -10 | 1 | -4 | -4 |

[Tables 12]

| % MIC of A alone | % MIC of B4 alone | MIC of each compound as a mixture | | FIC Index | Ratio A/B4 |
|---|---|---|---|---|---|
| | | A % | B4 % | | |
| 0.5 | 0.25 | 0.25 | 0.125 | 1 | 0.5 |

The results obtained show synergistic inhibitory activity for the mixtures:

i) 0.25 % of A and 0.125 % of B3, i.e. A/B4 ratio = 2

ii) 0.125 % of A and 0.125 % of B3, i.e. A/B4 ratio = 1

iii) 0.0625 % of A and 0.125 % of B3, i.e. A/B4 ratio = 0.5

**Example 5:**

The demonstration of a synergistic antimicrobial activity effect with a mixture of 4-(3-ethoxy-4-hydroxyphenyl) butan-2-one (referred to as substance A) and of 4-chloro-3,5-dimethylphenol (referred to as substance B5) was performed according to the conditions described in Example 1.

***Candida Albicans***

[Tables 13]

| **Concentrations tested (in weight %)** | 0 A | 0.025 A | 0.05 A | 0.1 A | **0.2 A** |
|---|---|---|---|---|---|
| 0 B5 | | 69 | 54 | 27 | 4 |
| 0.0625 B5 | 73 | 72 | 48 | 22 | 3 |
| 0.125 B5 | 28 | **15 (FIC 0.63)** | **11 (FIC 0.75)** | **6 (FIC 1)** | 1 |
| **0.25 B5** | 1 | 0 | 0 | 1 | 0 |

[Tables 14]

| % MIC of A alone | % MIC of B5 alone | MIC of each compound as a mixture | | FIC Index | Ratio A/B5 |
|---|---|---|---|---|---|
| | | A % | B5 % | | |
| 0.2 | 0.25 | 0.1 | 0.125 | 1 | 0.8 |

The results obtained show synergistic inhibitory activity for the mixtures:

0.1 % of A and 0.125 % of B5, i.e. A/B5 ratio = 0.8

0.05 % of A and 0.125 % of B, i.e. A/B5 ratio = 0.4

0.025 % of A and 0.125 % of B, i.e. A/B5 ratio = 0.2

*Aspergillus niger*

[Tables 15]

| Concentrations tested (in weight %) | 0 A | 0.025 A | 0.05 A | **0.1 A** |
|---|---|---|---|---|
| 0 B5 | | 55 | 32 | 16 |
| 0.0625 B5 | 99 | 31 | 21 | 13 |
| 0.125 B5 | 103 | 40 | **7 (FIC 0.75)** | 7 |
| 0.25 B5 | 31 | 48 | **2 (FIC 1)** | 7 |
| **0.5 B5** | -6 | -27 | -8 | -6 |

[Tables 16]

| % MIC of A alone | % MIC of B5 alone | MIC of each compound as a mixture | | FIC Index | Ratio A/B5 |
|---|---|---|---|---|---|
| | | A % | B5 % | | |
| 0.1 | 0.5 | 0.05 | 0.25 | 1 | 0.2 |

The results obtained show synergistic inhibitory activity for the mixtures:

i) 0.05 % of A and 0.25 % of B5, i.e. A/B5 ratio = 0.2

ii) 0.05 % of A and 0.125 % of B5, i.e. A/B5 ratio = 0.4

**Example 6:**

The demonstration of a synergistic antimicrobial activity effect with a mixture of 4-(3-ethoxy-4-hydroxyphenyl) butan-2-one (referred to as substance A) and of glyceryl caprylate (referred to as substance B6) was performed according to the conditions described in Example 1.

*Pseudomonas aeruginosa*

[Tables 17]

| Concentrations tested (in weight %) | 0 A | 0.0625 A | 0.125 A | 0.25 A | 0.5 A | **1 A** |
|---|---|---|---|---|---|---|
| 0 B6 | | 80 | 63 | 56 | 61 | -17 |
| 0.1 B6 | 123 | 84 | 58 | **18 (FIC 0.38)** | **11 (FIC 0.75)** | -7 |
| 0.2 B6 | 109 | 44 | **10 (FIC 0.63)** | **-5 (FIC 0.75)** | **-1 (FIC 1)** | -5 |
| **0.4 B6** | -40 | 12 | -5 | 2 | 1 | -2 |

[Tables 18]

| % MIC of A alone | % MIC of B6 alone | MIC of each compound as a mixture | | FIC Index | Ratio A/B6 |
|---|---|---|---|---|---|
| | | A % | B6 % | | |
| 1 | 0.4 | 0.5 | 0.2 | 1 | 2.5 |

The results obtained show synergistic inhibitory activity for the mixtures:

i) 0.5 % of A and 0.2 % of B6, i.e. A/B6 ratio = 2.5

ii) 0.25 % of A and 0.2 % of B6, i.e. A/B6 ratio = 1.25

iii) 0.125 % of A and 0.2 % of B6, i.e. A/B6 ratio = 0.625

iv) 0.5 % of A and 0.1 % of B6, i.e. A/B6 ratio = 5

v) 0.25 % of A and 0.1 % of B6, i.e. ratio A/B6 = 2.5

*Staphylococcus aureus*

[Tables 19]

| **Concentrations tested (in weight %)** | 0 A | 0.0625 A | 0.125 A | 0.25 A | 0.5 A | **1 A** |
|---|---|---|---|---|---|---|
| 0 B6 | | 65 | 75 | 79 | 24 | 5 |
| 0.0625 B6 | 83 | 80 | 27 | **-1 (FIC 0.31)** | **1 (FIC 0.56)** | -1 |
| 0.125 B6 | 87 | 58 | **0 (FIC 0.25)** | **1 (FIC 0.38)** | **-9 (FIC 0.63)** | 0 |
| 0.25 B6 | 53 | **-4 (FIC 0.31)** | **1 (FIC 0.38)** | **7 (FIC 0.5)** | **1 (FIC 0.75)** | 0 |
| 0.5 B6 | 48 | **0 (FIC 0.56)** | **-6 (FIC 0.63)** | **4 (FIC 0.75)** | **0 (FIC 1)** | -1 |
| **1 B6** | 9 | -1 | 0 | 6 | -3 | -6 |

[Tables 20]

| % MIC of A alone | % MIC of B6 alone | MIC of each compound as a mixture | | FIC Index | Ratio A/B6 |
|---|---|---|---|---|---|
| | | A % | B6 % | | |
| 1 | 1 | 0.5 | 0.5 | 1 | 1 |

The results obtained show synergistic inhibitory activity for the mixtures:

i) 0.5 % of A and 0.5 % of B6, i.e. A/B6 ratio = 1

ii) 0.25 % of A and 0.5 % of B6, i.e. A/B6 ratio = 0.5

iii) 0.125 % of A and 0.5 % of B6, i.e. A/B6 ratio = 0.25

iv) 0.0625 % of A and 0.5 % of B6, i.e. ratio A/B6 = 0.125

iv) 0.5 % of A and 0.25 % of B6, i.e. A/B6 ratio = 2

v) 0.25 % of A and 0.25 % of B6, i.e. ratio A/B6 = 1

vi) 0.125 % of A and 0.25 % of B6, i.e. ratio A/B6 = 0.5

vii) 0.0625 % of A and 0.25 % of B6, i.e. ratio A/B6 = 0.25

viii) 0.5 % of A and 0.125 % of B6, i.e. ratio A/B6 = 4

ix) 0.25 % of A and 0.125 % of B6, i.e. ratio A/B6 = 2

x) 0.125 % of A and 0.125 % of B6, i.e. ratio A/B6 = 1

xi) 0.5 % of A and 0.0625 % of B6, i.e. ratio A/B6 = 8

xii) 0.25 % of A and 0.0625 % of B6, i.e. ratio A/B6 = 4

***E. Faecalis***

[Tables 21]

| Concentrations tested (in weight %) | 0 A | 0.0625 A | 0.125 A | 0.25 A | **0.5 A** |
|---|---|---|---|---|---|
| 0 B6 | | 35 | 43 | 34 | 0 |
| 0.625 B6 | 38 | **16 (FIC 0.25)** | **12 (FIC 0.38)** | **4 (FIC 0.63)** | -4 |
| 0.125 B6 | 57 | **5 (FIC 0.38)** | **-1 (FIC 0.5)** | **-1 (FIC 0.75)** | -7 |
| 0.25 B6 | 22 | **-19 (FIC 0.63)** | **-12 (FIC 0.75)** | **-11 (FIC 1)** | 3 |
| **0.5 B6** | 8 | -3 | 1 | -24 | -7 |

[Tables 22]

| **% MIC of A alone** | **% MIC of B6 alone** | **MIC of each compound as a mixture** | | **FIC Index** | **Ratio A/B6** |
|---|---|---|---|---|---|
| | | **A %** | **B6 %** | | |
| 0.5 | 0.5 | 0.25 | 0.25 | 1 | 1 |

The results obtained show synergistic inhibitory activity for the mixtures:

i) 0.25 % of A and 0.25 % of B6, i.e. A/B6 ratio = 1

ii) 0.125 % of A and 0.25 % of B6, i.e. A/B6 ratio = 0.5

iii) 0.0625 % of A and 0.25 % of B6, i.e. A/B6 ratio = 0.25

iv) 0.25 % of A and 0.125 % of B6, i.e. ratio A/B6 = 2

v) 0.125 % of A and 0.125 % of B6, i.e. ratio A/B6 = 1

vi) 0.0625 % of A and 0.125 % of B6, i.e. ratio A/B6 = 0.5

vii) 0.25 % of A and 0.0625 % of B6, i.e. ratio A/B6 = 4

viii) 0.125 % of A and 0.0625 % of B6, i.e. ratio A/B6 = 2

ix) 0.0625 % of A and 0.0625 % of B6, i.e. ratio A/B6 = 1

## Claims

**1.** An antimicrobial mixture comprising :

- one or more 4-(3-ethoxy-4-hydroxyphenyl)butan-2-one and salts thereof with an organic or inorganic acid or base, and solvates thereof such as the hydrates ; and
- one or more alcohol compounds chosen from :
  - hydroxyacetophenone and salts thereof with an organic or inorganic base, and solvates thereof such as the hydrates;
  - 5-chloro-2-(2,4-dichlorophenoxy)phenol and salts thereof with an organic or inorganic base, and solvates thereof such as the hydrates;
  - sorbitan caprylate, and its optical isomers, and solvates thereof such as the hydrates;
  - the combination of 80 % to 90 % by weight, relative to the total weight of said combination, of xylityl sesquicaprylate and its optical isomers, and solvates thereof such as the hydrates and from 10% to 20% by weight of anhydroxylitol (especially 1,4-anhydroxylitol) and its optical isomers, and solvates thereof such as the hydrates;
  - 4-chloro-3,5-dimethylphenol and salts thereof with an organic or inorganic base, and solvates thereof such as the hydrates; and
  - glyceryl caprylate and its optical isomers, and solvates thereof such as the hydrates.

**2.** The mixture as claimed in the preceding claim, **characterized in that** the alcohol compound is hydroxyacetophenone and salts thereof with an organic or inorganic base, and solvates thereof such as the hydrates; preferably hydroxyacetophenone.

**3.** The mixture as claimed in either of the preceding claims, **characterized in that** it has a 4-(3-ethoxy-4-hydroxyphenyl) butan-2-one/hydroxyacetophenone weight ratio ranging from 0.08 to 0.5, preferably from 0.08 to 0.3, preferentially from 0.08 to 0.25, and more preferentially from 0.15 to 0.25.

**4.** The mixture as claimed in claim 1, **characterized in that** the alcohol compound is 5-chloro-2-(2,4-dichlorophenoxy) phenol and salts thereof with an organic or inorganic base and solvates thereof such as the hydrates, preferably 5-chloro-2-(2,4-dichlorophenoxy)phenol.

**5.** The mixture as claimed in the preceding claim, **characterized in that** it has a 4-(3-ethoxy-4-hydroxyphenyl)butan-2-one/5-chloro-2-(2,4-dichlorophenoxy)phenol weight ratio ranging from 0.3 to 6, preferably ranging from 0.7 to 6, preferentially ranging from 1.5 to 6 and more preferentially ranging from 3 to 6.

**6.** The mixture as claimed in claim 1, **characterized in that** the alcohol compound is sorbitan caprylate and its optical isomers, and solvates thereof such as the hydrates; preferably sorbitan caprylate.

**7.** The mixture as claimed in the preceding claim, **characterized in that** it has a 4-(3-ethoxy-4-hydroxyphenyl)butan-2-one/sorbitan caprylate weight ratio ranging from 0.1 to 3.2, preferably from 0.2 to 1.6 and preferably from 0.4 to 1.4.

8. The mixture as claimed in claim 6, **characterized in that** it has a 4-(3-ethoxy-4-hydroxyphenyl)butan-2-one/sorbitan caprylate weight ratio ranging from 0.4 to 1.6, preferably from 0.6 to 1.6 and preferably from 0.8 to 1.4.

9. The mixture as claimed in claim 6, **characterized in that** it has a 4-(3-ethoxy-4-hydroxyphenyl)butan-2-one/sorbitan caprylate weight ratio ranging from 0.1 to 3.2, preferably from 0.2 to 1.6 and preferably from 0.4 to 1.2.

10. The mixture as claimed in claim 1, **characterized in that** the alcohol compound is a combination of from 80% to 90% by weight, relative to the total weight of said combination, of xylityl sesquicaprylate and its optical isomers, and solvates thereof such as the hydrates; preferably xylityl sesquicaprylate and from 10% to 20% by weight of anhydroxylitol and its optical isomers, and solvates thereof such as the hydrates; preferably anhydroxylitol.

11. The mixture as claimed in the preceding claim, **characterized in that** it has a 4-(3-ethoxy-4-hydroxyphenyl)butan-2-one/combination of xylityl sesquicaprylate/anhydroxylitol weight ratio ranging from 0.2 to 13, preferably ranging from 0.2 to 10, preferably ranging from 0.2 to 8, more preferentially ranging from 0.4 to 5, preferably from 0.6 to 3.

12. The mixture as claimed in claim 10, **characterized in that** it has a 4-(3-ethoxy-4-hydroxyphenyl)butan-2-one/combination of xylityl sesquicaprylate/anhydroxylitol weight ratio ranging from 0.4 to 3.2, preferably from 0.6 to 3 and preferably from 1.5 to 2.5.

13. The mixture as claimed in claim 1, **characterized in that** the alcohol compound is 4-chloro-3,5-dimethylphenol and salts thereof with an organic or inorganic base, and solvates thereof such as the hydrates, especially 4-chloro-3,5-dimethylphenol.

14. The mixture as claimed in the preceding claim, **characterized in that** it has a 4-(3-ethoxy-4-hydroxyphenyl)butan-2-one/4-chloro-3,5-dimethylphenol weight ratio ranging from 0.1 to 1.4, preferably ranging from 0.15 to 1.4 and preferably ranging from 0.15 to 1.2.

15. The mixture as claimed in claim 13, **characterized in that** it has a 4-(3-ethoxy-4-hydroxyphenyl)butan-2-one/4-chloro-3,5-dimethylphenol weight ratio ranging from 0.15 to 1.4, preferably from 0.25 to 1.4 and preferably from 0.5 to 1.2.

16. The mixture as claimed in claim 13, **characterized in that** it has a 4-(3-ethoxy-4-hydroxyphenyl)butan-2-one/4-chloro-3,5-dimethylphenol weight ratio ranging from 0.1 to 0.7, preferably from 0.15 to 0.5, preferably from 0.15 to 0.35, preferably from 0.15 to 0.25, preferably from 0.3 to 0.7, preferably from 0.3 to 0.5.

17. The mixture as claimed in claim 1, **characterized in that** the alcohol compound is glyceryl caprylate and its optical isomers, and solvates thereof such as the hydrates; preferably glyceryl caprylate.

18. The mixture as claimed in the preceding claim, **characterized in that** it has a 4-(3-ethoxy-4-hydroxyphenyl)butan-2-one/glyceryl caprylate weight ratio ranging from 0.1 to 10.

19. The mixture as claimed in claim 17 or 18, **characterized in that** it has a 4-(3-ethoxy-4-hydroxyphenyl)butan-2-one/glyceryl caprylate weight ratio ranging from 0.5 to 9, preferably ranging from 0.5 to 7, preferably ranging from 1 to 6, preferably ranging from 1 to 3.

20. The mixture as claimed in claim 17 or 18, **characterized in that** it has a 4-(3-ethoxy-4-hydroxyphenyl)butan-2-one/glyceryl caprylate weight ratio ranging from 0.2 to 5, preferably ranging from 0.9 to 4.5.

21. A composition, preferably a cosmetic, in a physiologically acceptable medium, comprising :

- one or more 4-(3-ethoxy-4-hydroxyphenyl)butan-2-one and salts thereof with an organic or mineral base, and solvates thereof such as the hydrates as claimed in anyone of claims 1, 3, 5, 7 to 9, 11, 12, 14 to 16, and 18 to 20 ;
- one or more alcohol compounds chosen from :
  - hydroxyacetophenone and salts thereof with an organic or inorganic base, and solvates thereof such as the hydrates as claimed in anyone of claims 1, 2 or 3;
  - 5-chloro-2-(2,4-dichlorophenoxy)phenol and salts thereof with an organic or inorganic base, and solvates thereof such as the hydrates as claimed in anyone of claims 1, 4 or 5;

- sorbitan caprylate, and its optical isomers, and solvates thereof such as the hydrates as claimed in anyone of claims 1, 6 to 9;
- the combination of 80 % to 90 % by weight, relative to the total weight of said combination, of xylityl sesquicaprylate and its optical isomers, and solvates thereof such as the hydrates and from 10% to 20% by weight of anhydroxylitol (especially 1,4-anhydroxylitol) and its optical isomers, and solvates thereof such as the hydrates as claimed in anyone of claims 1, 10 to 12;
- 4-chloro-3,5-dimethylphenol and salts thereof with an organic or inorganic base, and solvates thereof such as the hydrates as claimed in anyone of claims 1, 13 to 16; and
- glyceryl caprylate and its optical isomers, and solvates thereof such as the hydrates as claimed in anyone of claims 1, 17 to 20.

**22.** A composition comprising, in a physiologically acceptable medium, an antimicrobial mixture as claimed in one of claims 1 to 20.

**23.** The composition as claimed in either of claims 21 and 22, **characterized in that** it comprises at least one additional ingredient chosen from water, oils, polyols containing from 2 to 10 carbon atoms, gelling agents, surfactants, film-forming polymers, dyestuffs, fragrances, fillers, UV-screening agents, plant extracts, cosmetic and dermatological active agents, and salts.

**24.** The composition as claimed in any one of the preceding claims 21 to 23, **characterized in that** the 4-(3-ethoxy-4-hydroxyphenyl)butan-2-one and salts thereof with an organic or mineral base, and solvates thereof such as the hydrates is present in a content ranging from 0.01% to 5% by weight relative to the total weight of the composition, preferably ranging from 0.01% to 3% by weight, preferentially ranging from 0.01% to 2.5% by weight, more preferentially ranging from 0.01% to 2% by weight and better still ranging from 0.02% to 0.15% by weight.

**25.** A nontherapeutic cosmetic treatment process for caring for and/or making up and/or cleansing keratin materials, comprising the application to said keratin materials of a composition as claimed in any one of claims 21 to 24.

**26.** A process for conserving a composition, comprising a physiologically acceptable medium, in particular a cosmetic or dermatological composition, **characterized in that** it consists in incorporating into said composition an antimicrobial mixture as defined in one of claims 1 to 20.

**27.** A process for preserving a nutritional composition, **characterized in that** it consists in incorporating into said composition an antimicrobial mixture as defined in one of claims 1 to 20.

**28.** The use of the antimicrobial mixture as defined in any one of claims 1 to 20, in the treatment of water, in which said water is chosen from domestic or industrial waters, waters from aquatic media, swimming pool/spa waters, and waters from air-conditioning systems.

**29.** A continuous or discontinuous water treatment process comprising at least one step of placing a water sample to be treated or a water stream to be treated, said water being chosen from domestic or industrial waters, waters from aquatic media, swimming pool/spa waters, and waters from air-conditioning systems, in contact with the antimicrobial mixture as defined in any one of claims 1 to 20.

**Patentansprüche**

**1.** Antimikrobielle Mischung, umfassend:

• ein oder mehrere 4-(3-Ethoxy-4-hydroxyphenyl)butan-2-on und Salze davon mit einer organischen oder anorganischen Säure oder Base und Solvate davon wie die Hydrate; und
• eine oder mehrere Alkoholverbindungen, ausgewählt aus:

- Hydroxyacetophenon und Salzen davon mit einer organischen oder anorganischen Base und Solvaten davon wie den Hydraten;
- 5-Chlor-2-(2,4-dichlorphenoxy)phenol und Salzen davon mit einer organischen oder anorganischen Base und Solvaten davon wie den Hydraten;
- Sorbitancaprylat und dessen optischen Isomeren und Solvaten davon wie den Hydraten;

- die Kombination von 80 bis 90 Gew.-%, bezogen auf das Gesamtgewicht der Kombination, Xylitylsesquicaprylat und dessen optischen Isomeren und Solvaten davon wie den Hydraten und 10 bis 20 Gew.-% Anhydroxylitol (insbesondere 1,4-Anhydroxylitol) und dessen optischen Isomeren und Solvaten davon wie den Hydraten;
- 4-Chlor-3,5-dimethylphenol und Salzen davon mit einer organischen oder anorganischen Base und Solvaten davon wie den Hydraten; und
- Glycerylcaprylat und dessen optischen Isomeren und Solvaten davon wie den Hydraten.

2. Mischung nach dem vorhergehenden Anspruch, **dadurch gekennzeichnet, dass** es sich bei der Alkoholverbindung um Hydroxyacetophenon und Salze davon mit einer organischen oder anorganischen Base sowie deren Solvate wie die Hydrate, vorzugsweise Hydroxyacetophenon, handelt.

3. Mischung nach einem der vorhergehenden Ansprüche, **dadurch gekennzeichnet, dass** sie ein Gewichtsverhältnis 4-(3-Ethoxy-4-hydroxyphenyl)butan-2-on/Hydroxyacetophenon im Bereich von 0,08 bis 0,5, vorzugsweise von 0,08 bis 0,3, bevorzugt von 0,08 bis 0,25 und besonders bevorzugt von 0,15 bis 0,25 aufweist.

4. Mischung nach Anspruch 1, **dadurch gekennzeichnet, dass** es sich bei der Alkoholverbindung um 5-Chlor-2-(2,4-dichlorphenoxy)phenol und Salze davon mit einer organischen oder anorganischen Base sowie deren Solvate wie die Hydrate, vorzugsweise 5-Chlor-2-(2,4-dichlorphenoxy)phenol, handelt.

5. Mischung nach dem vorhergehenden Anspruch, **dadurch gekennzeichnet, dass** sie ein Gewichtsverhältnis 4-(3-Ethoxy-4-hydroxyphenyl)butan-2-on/5-Chlor-2-(2,4-dichlorphenoxy)phenol im Bereich von 0,3 bis 6, vorzugsweise im Bereich von 0,7 bis 6, bevorzugt im Bereich von 1,5 bis 6 und weiter bevorzugt im Bereich von 3 bis 6 aufweist.

6. Mischung nach Anspruch 1, **dadurch gekennzeichnet, dass** es sich bei der Alkoholverbindung um Sorbitancaprylat und dessen optische Isomere und Solvate davon wie die Hydrate, vorzugsweise Sorbitancaprylat, handelt.

7. Mischung nach dem vorhergehenden Anspruch, **dadurch gekennzeichnet, dass** sie ein Gewichtsverhältnis 4-(3-Ethoxy-4-hydroxyphenyl)butan-2-on/Sorbitancaprylat im Bereich von 0,1 bis 3,2, vorzugsweise von 0,2 bis 1,6 und vorzugsweise von 0,4 bis 1,4 aufweist.

8. Mischung nach Anspruch 6, **dadurch gekennzeichnet, dass** sie ein Gewichtsverhältnis 4-(3-Ethoxy-4-hydroxyphenyl)butan-2-on/Sorbitancaprylat im Bereich von 0,4 bis 1,6, vorzugsweise von 0,6 bis 1,6 und vorzugsweise von 0,8 bis 1,4 aufweist.

9. Mischung nach Anspruch 6, **dadurch gekennzeichnet, dass** sie ein Gewichtsverhältnis 4-(3-Ethoxy-4-hydroxyphenyl)butan-2-on/Sorbitancaprylat im Bereich von 0,1 bis 3,2, vorzugsweise von 0,2 bis 1,6 und vorzugsweise von 0,4 bis 1,2 aufweist.

10. Mischung nach Anspruch 1, **dadurch gekennzeichnet, dass** es sich bei der Alkoholverbindung um eine Kombination von 80 bis 90 Gew.-%, bezogen auf das Gesamtgewicht der Kombination, Xylitylsesquicaprylat und dessen optischen Isomeren und Solvaten davon wie den Hydraten, vorzugsweise Xylitylsesquicaprylat, und 10 bis 20 Gew.-% Anhydroxylitol und dessen optischen Isomeren und Solvaten davon wie den Hydraten, vorzugsweise Anhydroxylitol, handelt;

11. Mischung nach dem vorhergehenden Anspruch, **dadurch gekennzeichnet, dass** sie ein Gewichtsverhältnis 4-(3-Ethoxy-4-hydroxyphenyl)butan-2-on/Kombination von Xylitsesquicaprylat/Anhydroxylitol im Bereich von 0,2 bis 13, vorzugsweise im Bereich von 0,2 bis 10, vorzugsweise im Bereich von 0,2 bis 8, weiter bevorzugt im Bereich von 0,4 bis 5, vorzugsweise von 0,6 bis 3, aufweist.

12. Mischung nach Anspruch 10, **dadurch gekennzeichnet, dass** sie ein Gewichtsverhältnis 4-(3-Ethoxy-4-hydroxyphenyl)butan-2-on/Kombination Xylitsesquicaprylat/Anhydroxylitol im Bereich von 0,4 bis 3,2, vorzugsweise von 0,6 bis 3 und vorzugsweise von 1,5 bis 2,5 aufweist.

13. Mischung nach Anspruch 1, **dadurch gekennzeichnet, dass** es sich bei der Alkoholverbindung um 4-Chlor-3,5-dimethylphenol und Salze davon mit einer organischen oder anorganischen Base sowie Solvate davon wie die Hydrate, insbesondere 4-Chlor-3,5-dimethylphenol, handelt.

**14.** Mischung nach dem vorhergehenden Anspruch, **dadurch gekennzeichnet, dass** sie ein Gewichtsverhältnis 4-(3-Ethoxy-4-hydroxyphenyl)butan-2-on/4-Chlor-3,5-dimethylphenol im Bereich von 0,1 bis 1,4, vorzugsweise im Bereich von 0,15 bis 1,4 und vorzugsweise im Bereich von 0,15 bis 1,2 aufweist.

**15.** Mischung nach Anspruch 13, **dadurch gekennzeichnet, dass** sie ein Gewichtsverhältnis 4-(3-Ethoxy-4-hydroxyphenyl)butan-2-on/4-Chlor-3,5-dimethylphenol im Bereich von 0,15 bis 1,4, vorzugsweise von 0,25 bis 1,4 und vorzugsweise von 0,5 bis 1,2 aufweist.

**16.** Mischung nach Anspruch 13, **dadurch gekennzeichnet, dass** sie ein Gewichtsverhältnis 4-(3-Ethoxy-4-hydroxyphenyl)butan-2-on/4-Chlor-3,5-dimethylphenol im Bereich von 0,1 bis 0,7, vorzugsweise von 0,15 bis 0,5, vorzugsweise von 0,15 bis 0,35, vorzugsweise von 0,15 bis 0,25, vorzugsweise von 0,3 bis 0,7, vorzugsweise von 0,3 bis 0,5, aufweist.

**17.** Mischung nach Anspruch 1, **dadurch gekennzeichnet, dass** es sich bei der Alkoholverbindung um Glycerylcaprylat und dessen optische Isomere und Solvate davon wie die Hydrate, vorzugsweise Glycerylcaprylat, handelt.

**18.** Mischung nach dem vorhergehenden Anspruch, **dadurch gekennzeichnet, dass** sie ein Gewichtsverhältnis 4-(3-Ethoxy-4-hydroxyphenyl)butan-2-on/Glycerylcaprylat im Bereich von 0,1 bis 10 aufweist.

**19.** Mischung nach Anspruch 17 oder 18, **dadurch gekennzeichnet, dass** sie ein Gewichtsverhältnis 4-(3-Ethoxy-4-hydroxyphenyl)butan-2-on/Glycerylcaprylat im Bereich von 0,5 bis 9, vorzugsweise im Bereich von 0,5 bis 7, vorzugsweise im Bereich von 1 bis 6, vorzugsweise im Bereich von 1 bis 3, aufweist.

**20.** Mischung nach Anspruch 17 oder 18, **dadurch gekennzeichnet, dass** sie ein Gewichtsverhältnis 4-(3-Ethoxy-4-hydroxyphenyl)butan-2-on/Glycerylcaprylat im Bereich von 0,2 bis 5, vorzugsweise im Bereich von 0,9 bis 4,5, aufweist.

**21.** Zusammensetzung, vorzugsweise ein Kosmetikum, in einem physiologisch unbedenklichen Medium, umfassend:

• ein oder mehrere 4-(3-Ethoxy-4-hydroxyphenyl)butan-2-on und Salze davon mit einer organischen Base oder Mineralbase und Solvate davon wie die Hydrate nach einem der Ansprüche 1, 3, 5, 7 bis 9, 11, 12, 14 bis 16 und 18 bis 20;
• eine oder mehrere Alkoholverbindungen, ausgewählt aus:

- Hydroxyacetophenon und Salze davon mit einer organischen oder anorganischen Base und Solvate davon wie die Hydrate nach einem der Ansprüche 1, 2 oder 3;
- 5-Chlor-2-(2,4-dichlorphenoxy)phenol und Salze davon mit einer organischen oder anorganischen Base und Solvate davon wie die Hydrate nach einem der Ansprüche 1, 4 oder 5;
- Sorbitancaprylat und seine optischen Isomere und Solvate davon wie die Hydrate nach einem der Ansprüche 1, 6 bis 9;
- die Kombination von 80 bis 90 Gew.-%, bezogen auf das Gesamtgewicht der Kombination, Xylitylsesquicaprylat und dessen optischen Isomeren und Solvaten davon wie den Hydraten und 10 bis 20 Gew.-% Anhydroxylitol (insbesondere 1,4-Anhydroxylitol) und dessen optischen Isomeren und Solvaten davon wie den Hydraten nach einem der Ansprüche 1, 10 bis 12;
- 4-Chlor-3,5-dimethylphenol und Salze davon mit einer organischen oder anorganischen Base und Solvate davon wie die Hydrate nach einem der Ansprüche 1, 13 bis 16; und
- Glycerylcaprylat und seine optischen Isomere und Solvate davon wie die Hydrate nach einem der Ansprüche 1, 17 bis 20.

**22.** Zusammensetzung, die in einem physiologisch unbedenklichen Medium eine antimikrobielle Mischung nach einem der Ansprüche 1 bis 20 umfasst.

**23.** Zusammensetzung nach Anspruch 21 oder 22, **dadurch gekennzeichnet, dass** sie mindestens einen zusätzlichen Bestandteil umfasst, der aus Wasser, Ölen, Polyolen mit 2 bis 10 Kohlenstoffatomen, Geliermitteln, Tensiden, filmbildenden Polymeren, Farbstoffen, Duftstoffen, Füllstoffen, UV-Filtersubstanzen, Pflanzenextrakten, kosmetischen und dermatologischen Wirkstoffen und Salzen ausgewählt ist.

**24.** Zusammensetzung nach einem der vorhergehenden Ansprüche 21 bis 23, **dadurch gekennzeichnet, dass** das

4-(3-Ethoxy-4-hydroxyphenyl)butan-2-on und Salze davon mit einer organischen Base oder Mineralbase und Solvate davon wie die Hydrate in einem Gehalt im Bereich von 0,01 bis 5 Gew.-%, bezogen auf das Gesamtgewicht der Zusammensetzung, vorzugsweise im Bereich von 0,01 bis 3 Gew.-%, bevorzugt im Bereich von 0,01 bis 2,5 Gew.-%, weiter bevorzugt im Bereich von 0,01 bis 2 Gew.-% und noch besser im Bereich von 0,02 bis 0,15 Gew.-% vorliegt.

**25.** Nichttherapeutisches kosmetisches Behandlungsverfahren zum Pflegen und/oder Schminken und/oder Reinigen von Keratinmaterialien, umfassend das Aufbringen einer Zusammensetzung nach einem der Ansprüche 21 bis 24 auf die Keratinmaterialien.

**26.** Verfahren zum Konservieren einer Zusammensetzung, die ein physiologisch unbedenkliches Medium umfasst, insbesondere einer kosmetischen oder dermatologischen Zusammensetzung, **dadurch gekennzeichnet, dass** es aus dem Einarbeiten einer antimikrobiellen Mischung gemäß einem der Ansprüche 1 bis 20 in die Zusammensetzung besteht.

**27.** Verfahren zum Konservieren einer Ernährungszusammensetzung, **dadurch gekennzeichnet, dass** es aus dem Einarbeiten einer antimikrobiellen Mischung gemäß einem der Ansprüche 1 bis 20 in die Zusammensetzung besteht.

**28.** Verwendung der antimikrobiellen Mischung gemäß einem der Ansprüche 1 bis 20 bei der Behandlung von Wasser, wobei das Wasser aus Haushalts- oder Industriewässern, Wässern aus aquatischen Medien, Schwimmbad-/Heilbad-Wässern und Wässern aus Klimaanlagen ausgewählt ist.

**29.** Kontinuierliches oder diskontinuierliches Wasserbehandlungsverfahren, umfassend mindestens einen Schritt des Inkontaktbringens einer zu behandelnden Wasserprobe oder eines zu behandelnden Wasserstroms, wobei das Wasser aus Haushalts- oder Industriewässern, Wässern aus aquatischen Medien, Schwimmbad-/Heilbad-Wässern und Wässern aus Klimaanlagen ausgewählt wird, mit der antimikrobiellen Mischung gemäß einem der Ansprüche 1 bis 20.

## Revendications

**1.** Mélange antimicrobien comprenant :

- une ou plusieurs 4-(3-éthoxy-4-hydroxyphényl)butan-2-one et ses sels avec un acide ou une base organique ou inorganique, et ses solvates tels que les hydrates ; et
- un ou plusieurs composés alcools choisis parmi :

  - hydroxyacétophénone et ses sels avec une base organique ou inorganique, et ses solvates tels que les hydrates ;
  - 5-chloro-2-(2,4-dichlorophénoxy)phénol et ses sels avec une base organique ou inorganique, et ses solvates tels que les hydrates ;
  - caprylate de sorbitane et ses isomères optiques, et ses solvates tels que les hydrates ;
  - la combinaison de 80 % à 90 % en poids, par rapport au poids total de ladite combinaison, de sesquicaprylate de xylityle et de ses isomères optiques, et de ses solvates tels que les hydrates et de 10 % à 20 % en poids d'anhydroxylitol (notamment de 1,4-anhydroxylitol) et de ses isomères optiques, et de ses solvates tels que les hydrates ;
  - 4-chloro-3,5-diméthylphénol et ses sels avec une base organique ou inorganique, et ses solvates tels que les hydrates ; et
  - caprylate de glycéryle et ses isomères optiques, ainsi que ses solvates tels que les hydrates.

**2.** Mélange selon la revendication précédente, **caractérisé en ce que** le composé alcool est l'hydroxyacétophénone et ses sels avec une base organique ou inorganique, et ses solvates tels que les hydrates ; de préférence l'hydroxyacétophénone.

**3.** Mélange selon l'une quelconque des revendications précédentes, **caractérisé en ce qu'**il présente un rapport pondéral 4-(3-éthoxy-4-hydroxyphényl)butan-2-one/hydroxyacétophénone allant de 0,08 à 0,5, de préférence de 0,08 à 0,3, préférentiellement de 0,08 à 0,25, et plus préférentiellement de 0,15 à 0,25.

**4.** Mélange selon la revendication 1, **caractérisé en ce que** le composé alcool est le 5-chloro-2-(2,4-dichlorophénoxy)phénol et ses sels avec une base organique ou inorganique et ses solvates tels que les hydrates, de préférence le 5-chloro-2-(2,4-dichlorophénoxy)phénol.

5. Mélange selon la revendication précédente, **caractérisé en ce qu'**il présente un rapport pondéral 4-(3-éthoxy-4-hydroxyphényl)butan-2-one/5-chloro-2-(2,4-dichlorophénoxy)phénol allant de 0,3 à 6, de préférence allant de 0,7 à 6, préférentiellement allant de 1,5 à 6 et plus préférentiellement allant de 3 à 6.

6. Mélange selon la revendication 1, **caractérisé en ce que** le composé alcool est le caprylate de sorbitane et ses isomères optiques, et ses solvates tels que les hydrates ; de préférence le caprylate de sorbitane.

7. Mélange selon la revendication précédente, **caractérisé en ce qu'**il présente un rapport pondéral 4-(3-éthoxy-4-hydroxyphényl)butan-2-one/caprylate de sorbitane allant de 0,1 à 3,2, de préférence de 0,2 à 1,6 et de préférence de 0,4 à 1,4.

8. Mélange selon la revendication 6, **caractérisé en ce qu'**il présente un rapport pondéral 4-(3-éthoxy-4-hydroxyphényl)butan-2-one/caprylate de sorbitane allant de 0,4 à 1,6, de préférence de 0,6 à 1,6 et de préférence de 0,8 à 1,4.

9. Mélange selon la revendication 6, **caractérisé en ce qu'**il présente un rapport pondéral 4-(3-éthoxy-4-hydroxyphényl)butan-2-one/caprylate de sorbitane allant de 0,1 à 3,2, de préférence de 0,2 à 1,6 et de préférence de 0,4 à 1,2.

10. Mélange selon la revendication 1, **caractérisé en ce que** le composé alcool est une combinaison de 80 % à 90 % en poids, par rapport au poids total de ladite combinaison, de sesquicaprylate de xylityle et de ses isomères optiques, et de ses solvates tels que les hydrates ; de préférence de sesquicaprylate de xylityle et de 10 % à 20 % en poids d'anhydroxylitol et de ses isomères optiques, et de ses solvates tels que les hydrates ; de préférence l'anhydroxylitol.

11. Mélange selon la revendication précédente, **caractérisé en ce qu'**il présente un rapport pondéral 4-(3-éthoxy-4-hydroxyphényl)butan-2-one/combinaison sesquicaprylate de xylityle/anhydroxylitol allant de 0,2 à 13, de préférence allant de 0,2 à 10, de préférence allant de 0,2 à 8, plus préférentiellement allant de 0,4 à 5, de préférence de 0,6 à 3.

12. Mélange selon la revendication 10, **caractérisé en ce qu'**il présente un rapport pondéral 4-(3-éthoxy-4-hydroxyphényl)butan-2-one/combinaison sesquicaprylate de xylityle/anhydroxylitol allant de 0,4 à 3,2, de préférence de 0,6 à 3 et de préférence de 1,5 à 2,5.

13. Mélange selon la revendication 1, **caractérisé en ce que** le composé alcool est le 4-chloro-3,5-diméthylphénol et ses sels avec une base organique ou inorganique, et ses solvates tels que les hydrates, en particulier le 4-chloro-3,5-diméthylphénol.

14. Mélange selon la revendication précédente, **caractérisé en ce qu'**il présente un rapport pondéral 4-(3-éthoxy-4-hydroxyphényl)butan-2-one/4-chloro-3,5-diméthylphénol allant de 0,1 à 1,4, de préférence allant de 0,15 à 1,4 et de préférence allant de 0,15 à 1,2.

15. Mélange selon la revendication 13, **caractérisé en ce qu'**il présente un rapport pondéral 4-(3-éthoxy-4-hydroxyphényl)butan-2-one/4-chloro-3,5-diméthylphénol allant de 0,15 à 1,4, de préférence de 0,25 à 1,4 et de préférence de 0,5 à 1,2.

16. Mélange selon la revendication 13, **caractérisé en ce qu'**il présente un rapport pondéral 4-(3-éthoxy-4-hydroxyphényl)butan-2-one/4-chloro-3,5-diméthylphénol allant de 0,1 à 0,7, de préférence de 0,15 à 0,5, de préférence de 0,15 à 0,35, de préférence de 0,15 à 0,25, de préférence de 0,3 à 0,7, de préférence de 0,3 à 0,5.

17. Mélange selon la revendication 1, **caractérisé en ce que** le composé alcool est le caprylate de glycéryle et ses isomères optiques, et ses solvates tels que les hydrates ; de préférence le caprylate de glycéryle.

18. Mélange selon la revendication précédente, **caractérisé en ce qu'**il présente un rapport pondéral 4-(3-éthoxy-4-hydroxyphényl)butan-2-one/caprylate de glycéryle allant de 0,1 à 10.

19. Mélange selon la revendication 17 ou 18, **caractérisé en ce qu'**il présente un rapport pondéral 4-(3-éthoxy-4-hydroxyphényl)butan-2-one/caprylate de glycéryle allant de 0,5 à 9, de préférence allant de 0,5 à 7, de préférence allant de 1 à 6, de préférence allant de 1 à 3.

**20.** Mélange selon la revendication 17 ou 18, **caractérisé en ce qu'**il présente un rapport pondéral 4-(3-éthoxy-4-hydroxyphényl)butan-2-one/caprylate de glycéryle allant de 0,2 à 5, de préférence allant de 0,9 à 4,5.

**21.** Composition, de préférence produit cosmétique, dans un milieu physiologiquement acceptable, comprenant :

• une ou plusieurs 4-(3-éthoxy-4-hydroxyphényl)butan-2-one et ses sels avec une base organique ou minérale, et ses solvates tels que les hydrates selon l'une quelconque des revendications 1, 3, 5, 7 à 9, 11, 12, 14 à 16 et 18 à 20 ;
• un ou plusieurs composés alcools choisis parmi :

- hydroxyacétophénone et ses sels avec une base organique ou inorganique, et ses solvates tels que les hydrates selon l'une quelconque des revendications 1, 2 ou 3 ;
- 5-chloro-2-(2,4-dichlorophénoxy)phénol et ses sels avec une base organique ou inorganique, et ses solvates tels que les hydrates selon l'une quelconque des revendications 1, 4 ou 5 ;
- caprylate de sorbitane, et ses isomères optiques, et ses solvates tels que les hydrates selon l'une quelconque des revendications 1, 6 à 9 ;
- la combinaison de 80 % à 90 % en poids, par rapport au poids total de ladite combinaison, de sesquicaprylate de xylityle et de ses isomères optiques, et de ses solvates tels que les hydrates et de 10 % à 20 % en poids d'anhydroxylitol (notamment de 1,4-anhydroxylitol) et de ses isomères optiques, et de ses solvates tels que les hydrates selon l'une quelconque des revendications 1, 10 à 12 ;
- 4-chloro-3,5-diméthylphénol et ses sels avec une base organique ou inorganique, et ses solvates tels que les hydrates selon l'une quelconque des revendications 1, 13 à 16 ; et
- caprylate de glycéryle et ses isomères optiques, ainsi que ses solvates tels que les hydrates selon l'une quelconque des revendications 1, 17 à 20.

**22.** Composition comprenant, dans un milieu physiologiquement acceptable, un mélange antimicrobien selon l'une des revendications 1 à 20.

**23.** Composition selon l'une des revendications 21 ou 22, **caractérisée en ce qu'**elle comprend au moins un ingrédient supplémentaire choisi parmi l'eau, les huiles, les polyols contenant de 2 à 10 atomes de carbone, les agents gélifiants, les tensioactifs, les polymères filmogènes, les colorants, les fragrances, les charges, les filtres UV, les extraits végétaux, les agents actifs cosmétiques et dermatologiques, et les sels.

**24.** Composition selon l'une quelconque des revendications précédentes 21 à 23, **caractérisée en ce que** la 4-(3-éthoxy-4-hydroxyphényl)butan-2-one et ses sels avec une base organique ou minérale, et ses solvates tels que les hydrates est présente en une teneur allant de 0,01 à 5 % en poids par rapport au poids total de la composition, de préférence allant de 0,01 à 3 % en poids, préférentiellement allant de 0,01 à 2,5 % en poids, plus préférentiellement allant de 0,01 à 2 % en poids et mieux encore allant de 0,02 à 0,15 % en poids.

**25.** Procédé de traitement cosmétique non thérapeutique pour le soin et/ou le maquillage et/ou le nettoyage de matières kératiniques, comprenant l'application sur lesdites matières kératiniques d'une composition selon l'une quelconque des revendications 21 à 24.

**26.** Procédé de conservation d'une composition, comprenant un milieu physiologiquement acceptable, en particulier une composition cosmétique ou dermatologique, **caractérisé en ce qu'**il consiste à incorporer dans ladite composition un mélange antimicrobien tel que défini dans l'une des revendications 1 à 20.

**27.** Procédé de conservation d'une composition nutritionnelle, **caractérisé en ce qu'**il consiste à incorporer dans ladite composition un mélange antimicrobien tel que défini dans l'une des revendications 1 à 20.

**28.** Utilisation du mélange antimicrobien tel que défini dans l'une quelconque des revendications 1 à 20, dans le traitement de l'eau, dans laquelle ladite eau est choisie parmi les eaux domestiques ou industrielles, les eaux de milieux aquatiques, les eaux de piscines/spas, et les eaux de systèmes de climatisation.

**29.** Procédé de traitement d'eau en continu ou discontinu comprenant au moins une étape de mise en contact d'un échantillon d'eau à traiter ou d'un flux d'eau à traiter, ladite eau étant choisie parmi les eaux domestiques ou industrielles, les eaux de milieux aquatiques, les eaux de piscines/spas et les eaux de systèmes de climatisation, avec le mélange antimicrobien tel que défini dans l'une quelconque des revendications 1 à 20.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011039445 A


### Non-patent literature cited in the description


- *CHEMICAL ABSTRACTS*, 95508-00-2
- *CHEMICAL ABSTRACTS*, 181632-90-6
- *CHEMICAL ABSTRACTS*, 53448-53-6
- *CHEMICAL ABSTRACTS*, 26402-26-6
- Fungal Contaminants in drinking water regulation? A tale of ecology, exposure, purification and clinical relevance. Int. J. Environ. Res. Public Health, 2017, vol. 14, 636
- **MICHAEL S. GILMORE et al.** Enterococci, from commensals to leading causes of drug resistant infection. Massachusetts Eye and Ear Infirmary, 2014
- **F.C. KULL** ; **P.C. EISMAN** ; **H.D. SYLWESTROWKA** ; **R.L. MAYER**. *Applied Microbiology*, 1961, vol. 9, 538-541